Europäisches Patentamt

European Patent Office

Office européen des brevets

(19)

(11) **EP 0 957 756 B1**

(12) **EUROPEAN PATENT SPECIFICATION**

(45) Date of publication and mention
of the grant of the patent:
**31.03.2004 Bulletin 2004/14**

(51) Int Cl.$^7$: **A61B 5/021**, A61B 5/026,
G06F 17/00

(21) Application number: **96905600.1**

(22) Date of filing: **15.03.1996**

(86) International application number:
**PCT/AU1996/000148**

(87) International publication number:
**WO 1996/029004 (26.09.1996 Gazette 1996/43)**

(54) **NON-INVASIVE DETERMINATION OF AORTIC FLOW VELOCITY WAVEFORMS**

NICHT-INVASIVE BESTIMMUNG DER FORM VON
STRÖMUNGSGESCHWINDIGKEITSSIGNALEN IN DER AORTA

DETERMINATION NON INVASIVE DES FORMES D'ONDES DU DEBIT AORTIQUE

(84) Designated Contracting States:
**AT BE CH DE ES FR GB IT LI NL SE**

(30) Priority: **17.03.1995 AU PN017989**

(43) Date of publication of application:
**24.11.1999 Bulletin 1999/47**

(73) Proprietor: **AtCor Medical Pty. Ltd.**
**West Ryde, NSW 2114 (AU)**

(72) Inventor: **O'ROURKE, Michael, Francis**
**Unters Hill, NSW 2110 (AU)**

(74) Representative: **Wilson, Peter et al**
**William Jones Limited**
**Willow Lane House**
**Willow Lane**
**Norwich**
**Norfolk NR2 1EU (GB)**

(56) References cited:
**EP-A- 0 487 726**         **WO-A-88/01773**
**US-A- 5 101 828**         **US-A- 5 289 823**

## Description

### Technical Field

The present invention relates to the derivation of aortic flow waveforms, using non-invasive techniques, in particular, measurement of pressure waveforms in peripheral arteries.

### Background Art

It is desirable in a wide range of clinical applications to be able to assess the flow velocity waveform in the ascending aorta. Flow velocity may be defined as average volume per second per unit area of the vessel. Flow velocity may be converted to actual volume per second if the vessel's internal diameter is known or determined. For the purposes of this determination, the velocity profile across the vessel may be assumed to be flat - we are here concerned with overall volume flow and rate of flow.

Flow velocity data is useful, for example, in assessing patients presenting with symptoms of cardiac disease, hypertension, and angina pectoris, and in following the response of these patients to treatment. However, techniques in use for assessing these parameters have not been appropriate for routine use. One known invasive technique utilises a probe inserted into an artery. It is also possible to utilise ultrasonic echo flow techniques, however, this has the drawback of using relatively expensive and complex equipment, and requiring a very high level of skill on the part of the operator to produce reliable results.

In a paper, " Computation of aortic flow from pressure in humans using a non-linear, three element model", J.Appl.Physiol.74(5):2566-2573, 1993, Wesseling et al disclose a method for computing aortic flow from radial pressure waveforms. The calculations described use a Windkessel type model, and whilst some account is taken of age, no account is taken of wave reflection, the timing of wave reflection, nor the changes in wave reflection or impedance which occur with age.

In a paper by Fry DL, " The measurement of pulsatile blood flow by the computed pressure gradient technique", IREE Transactions on Medical Electronics 6:259-264, 1959, an analog processing arrangement was used to produce a derived flow wave, with criteria imposed relating to the observed characteristics of the system. In particular, the flow wave at the incisura (identified by a flag) was required to approach zero, or pass from positive to negative within 10 ms of the incisura, and flow during diastole is required to be zero or within 3% of zero compared to peak systolic flow, and to show no systematic increase or decrease during diastole.

US 5289823 discloses a method for non-invasively measuring aortic blood flow. Sensors detect and produce arterial blood pressure signals. These signals are subjected to further processing prior to final display by applied transfer functions. These functions may be tailored to the individual patient based on age, weight and/or other factors.

### SUMMARY OF THE INVENTION

The present invention provides a method for determining an aortic flow velocity waveform, comprising the steps of:

a) measuring non-invasively at a peripheral site a blood pressure pulse waveform;
b) determining a calibrated substantially simultaneous systolic and diastolic pressure at the peripheral site, and thereby calibrating the waveform of (a);
c) calculating the calibrated aortic pressure pulse waveform at the ascending aorta using a predetermined transfer function;
d) calculating the aortic flow velocity waveform from said aortic pressure pulse waveform by reference to predetermined values for age dependent impedance modulus and phase; and
e) determining whether the waveform calculated at (d) meets predefined criteria, and if it does not, then repeating step (d) iteratively using different values for age until said waveform meets said predefined criteria.

Preferably, said predefined criteria include requirements that at the time of incisura, (that is, during diastole) the flow is substantially zero, and that after incisura flow remains within a predetermined margin, say 3 % of peak flow relative to zero.

The latter requirement seeks to ensure that computed values correspond to the reality that after incisura, the aortic valve is shut and there is no driving pressure, and accordingly apart from minor effects no positive or negative flow will occur.

Step d is preferably performed by performing Fourier analysis on the derived pressure waveform, calculating the flow wave components corresponding to each frequency component of the pressure wave separately, and combining the resulting waveforms to provide a derived flow velocity waveform.

It will be understood that the present invention may be applied to produce an uncalibrated waveform, which may be of use in some situations, although the calibrated version is preferred.

The values for modulus and phase are preferably as described hereinafter, however, it will be appreciated that such values may alternatively be determined by conducting appropriate further clinical studies.

The present invention provides a relatively simple, non-invasive procedure for determining calibrated velocity flow waveforms in humans, which takes account of variations of impedance and phase with age. Moreover, by performing an iteration of age to ensure the output derived waveform matches real parameters, a separate indication of the apparent "age" of the vascular system of the patient can be provided, which may in itself be of clinical relevance.

**Brief Description of Drawings**

An embodiment of the present invention will now be described with reference to the accompanying figures, in which:

Figure 1 illustrates ascending aortic impedance modulus as a function of frequency for ages 20 and 80;
Figure 2 illustrates ascending aortic impedance phase, as a function of frequency, for ages 20 and 80; and
Figure 3 illustrates graphically the calculation process for the derived flow velocity waveform according to a preferred calculation technique.

**Description**

The present invention relies on the use of an ascending aortic pressure waveform derived from a peripheral pressure waveform, and so initially this process will be briefly described. This procedure is discussed in more detail in US Patent No. 5265011 to O'Rourke, in a paper, "An analysis of the relationship between central aortic and peripheral upper limb pressure waves in Man", Karamanoglu, O'Rourke, Avolio and Kelly, European Heart Journal (1993) **14**, 160-167, and in the texts "The Arterial Pulse", O'Rourke, Kelly and Avolio, published by Lea Febiger, Philadelphia 1992 and "Arterial Vasodilation", O'Rourke, Saffer, Dzau, published by Arnold, London 1993. Reference should be made to these documents if clarification is required of this part of the process.

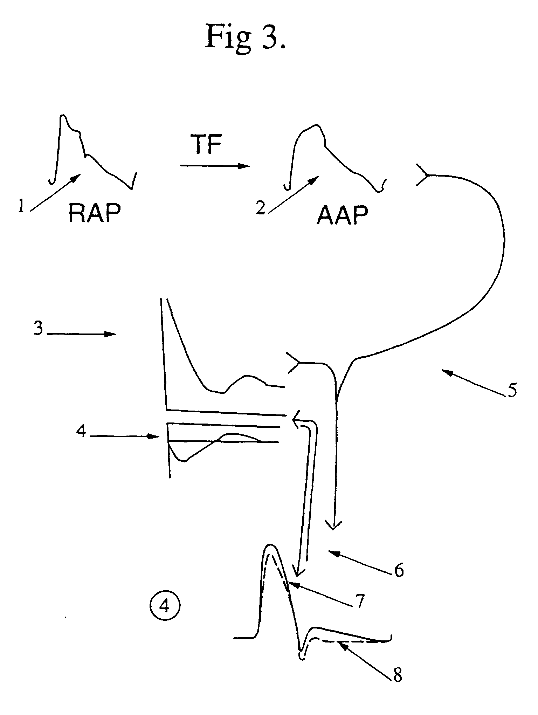

Briefly, the pressure waveforms of the peripheral arteries can be related to the ascending aortic waveform, by means of a clinically determined transfer function. This transfer function is most readily applied by deriving the Fourier transform of the measured waveform, applying the transfer function to each component sinusoid, and combining the calculated values. The measured waveform may be calibrated by measuring the systolic and diastolic pressures, using conventional sphygmomanometry, at a site comparable to the waveform measurement site. For example, it is convenient to measure waveforms at the radial artery, and to calibrate this from the brachial artery. Figure 3 shows a sample waveform 2 for the ascending aortic pressure, labelled as AAP. This has been derived from the radial waveform RAP, using transfer function TF. It will be appreciated, however, that the present invention is not limited in scope to utilising the exact procedures and transfer functions of the O'Rourke patent to derive the AAP waveform.

The above cited O'Rourke patent further describes a technique for the automatic determination of the point of incisura, using the third derivative of the measured peripheral pulse, by locating a zero crossing from positive to negative in proximity to the largest maximum point of the third derivative after the peak of a second systolic shoulder. However, it is contemplated that an implementation may allow for the point of incisura to be manually overridden by the physician based upon his interpretation of the waveform. An alternative method for calculating or otherwise determining the point of incisura may be utilised within the present invention if desired. The point of incisura is particularly relevant to the preferred implementation, as will be described below.

It will be understood that the calculation and processing described below may be readily implemented in a microprocessor type device using well understood software techniques, most conveniently in a laptop or other portable computer. Any suitable calculating and processing device may be used to implement the inventive techniques.

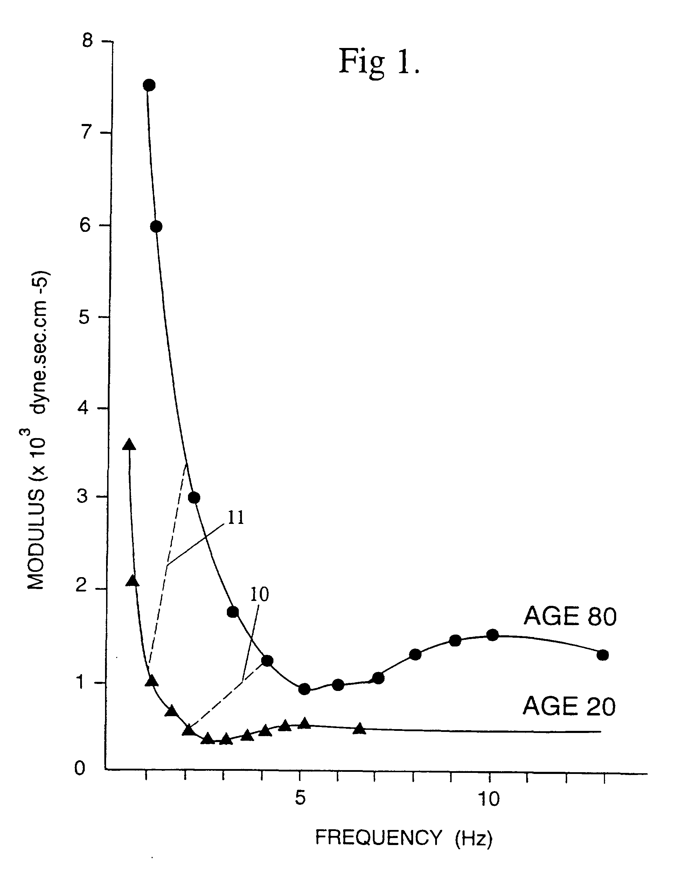

Figure 1 shows a plot of modulus against frequency, for ages 20 and 80. Although if desired a more elaborate set of interpolations could be used to implement the present invention, a reasonable value of modulus for intervening ages can be determined by an interpolation procedure as will be described below. Fully grown subjects under 20 may be assumed to be 20; over 80 may be assumed to be 80. The modulus may be considered as a linear impedance value, which can be applied at the frequency of each frequency component of the Fourier transform of the derived aortic pressure pulse. The amplitude of each frequency component is determined by dividing by the respective modulus value.

Considering the curves in figure 1, it has been determined clinically that the general trend of the curves with age is that an equivalent point for any given frequency on the age 20 curve corresponds to a point at twice that frequency on the age 80 curve, and this relationship can be used to provide a basis for interpolation.

Hence, interpolation may be performed by taking points at, for example, 0.5 Hz intervals on the age 20 curve, taking points on the age 80 curve at twice the frequency of the corresponding age 20 points, and drawing a line between the curves. Ilustrative interpolation lines are shown on figure 1 as lines 10 and 11. Along each line, a point can be marked at a fraction of the distance along the line from the age 20 curve corresponding to the modulus curve at a given intervening age X using the relation:

$$F_x = (X-20)/60$$

to give the fraction $F_x$ required along each line. For example, for a 35 year old, the fraction along each line is 0.25 along each line measured from the age 20 point.

It will be appreciated that if further clinical data is available for intervening ages, then a similar process could be used between each curve for which data was available. It is preferred that this aspect be implemented as a look-up table in a device. It will be understood that it is only the modulus value of each age curve which is required at discrete frequency component values, not the entire curve. Hence, it will be appreciated that an entire set of curves could be readily calculated and the values at suitable intervals stored in a look up table for use during automatic calculation.

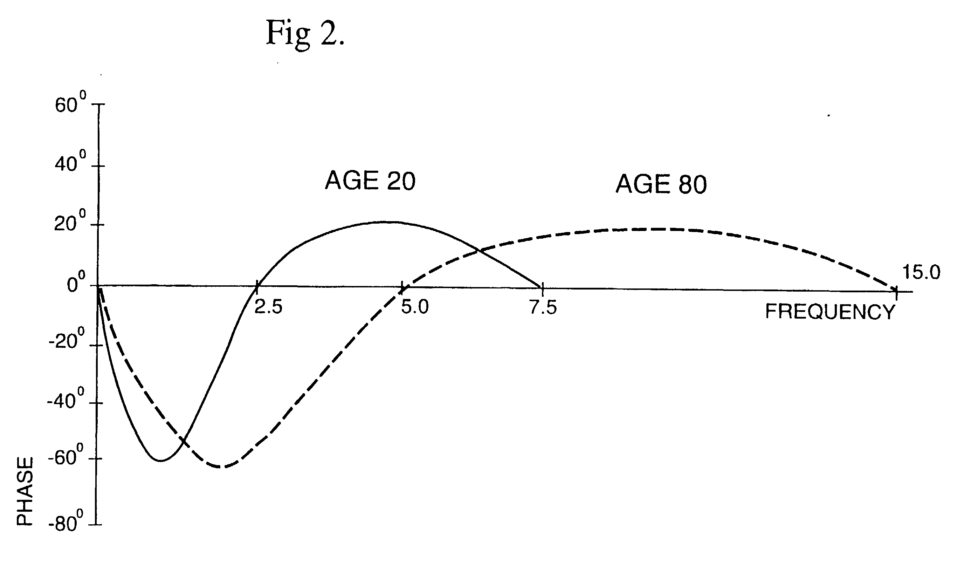

Figure 2 illustrates the phase difference between the pressure and flow waves in the ascending aorta, at different frequencies, once again for ages 20 and 80. It will be appreciated that as a person ages, compliance is reduced, and so the pattern of flow propagation resulting from a given pressure stimulus is different. Again, for ages between 20 and 80, a linear interpolation can be performed to derive the corresponding phase diagrams for intermediate ages. Again, it is preferred that the values be stored for suitable frequency and age values in a look up table.

Figure 3 illustrates the calculation process graphically. The calibrated ascending aortic pulse waveform AAP 2 is derived from the calibrated radial artery waveform 1, as previously described. The resulting waveform is then subjected to Fourier analysis. The lower frequency component is, in general, dominant, and useful results can be derived by utilising only a limited number, for example the first 8 to 10 frequency components. Of course, if more frequency components are used, the overall accuracy will be improved, particularly if it is desired to characterise the short negative flow period typically occurring after the incisura.

For each frequency component, the age related modulus value for the sinusoid's frequency from figure 1, referenced here as 3, is used to determine the amplitude of the corresponding flow wave component, and the phase difference from figure 2, here referenced as 4, is used to define the phase of the corresponding component. At this stage, an input value for age, being either the patient's actual age or the physician's estimated alternative value, is used. The resulting waveforms are combined, to produce an output flow waveform 7.

In a preferred implementation, this waveform is then tested against criteria which any flow waveform of this type must possess. One such characteristic is that after incisura, the time of which is known from the RAP to AAP process, the flow waveform must be zero. A second is that after incisura, the waveform must remain within a defined margin, for example 3%, of peak flow. Other criteria could be used in addition if desired. This could be performed manually, or within the processor of the present invention.

If the resulting waveform does not meet these criteria, or correspond within defined limits, then a process of iteration is commenced, in which the "age" value is varied up and down, and the flow waveform recalculated, until a best fit waveform 8 is determined. Ideally, this imputed age is available by display or otherwise to the physician. Although not necessarily determinative of any condition, the necessity to impute an age of, say, 60 to obtain a best fit for a man whose chronological age is 45 may be of clinical relevance.

Much of the process of calculation required to be implemented according to a preferred implementation in software differs only in detail from the existing software used to implement the derivation of pressure waveforms at the ascending aorta from the radial waveform, and so no detail will be provided. From the ascending calculation of the ascending aorta waveform, the fourier transform of this waveform in already known. For each frequency component, a lookup table is used to provide the phase and modulus values for the age or imputed age of the patient, and the fourier transform of the flow waveform is derived. This is converted to the time domain waveform. If the predefined criteria mentioned above are not met, the process is repeated using successive age values until the best fit is found. This type of software process is well understood by those skilled in the art.

It will be appreciated that while the present invention is directly concerned with determining the flow waveform, as this is a calibrated measurement it can be used to calculate the volume rate of flow. A value for the diameter, and hence area, of the aorta may be obtained from standard tables or formulae, or may have been obtained clinically, for example from echocardiography.

It will be appreciated that more elaborate impedance modulus and phase values, including other factors such as sex, or more detailed intermediate measurements, could be utilised. Additionally, further factors may be used in the transfer functions to derive the AAP waveforms, for example age, height and blood pressure. In comparison to their influence on impedance, these effects on transfer function for pressure are small.

**Claims**

1. A method for determining an aortic flow velocity waveform, including the steps of:

   a. measuring non-invasively at a peripheral site a blood pressure pulse waveform;
   b. determining a calibrated substantially simultaneous systolic and diastolic pressure at the peripheral site;
   c. calculating the calibrated aortic pressure pulse waveform at the ascending aorta using a predetermined transfer function; **characterized by** further including the steps of:
   d. calculating the aortic flow velocity waveform from said aortic pressure pulse waveform by reference to predetermined values for age dependent impedance modulus and phase; and

e. determining whether the waveform calculated at (d) meets predefined criteria, and if it does not, then repeating step (d) iteratively using different values for age until said waveform meets said predefined criteria.

2. A method according to claim 1, wherein said method includes storing an input or calculated time value for the point of incisura for said calibrated aortic pressure pulse waveform, and said predefined criteria include that at the point of incisura, the flow is substantially zero.

3. A method according to claim 2, wherein said predefined criteria include that after incisura flow remains within a predetermined margin of peak flow relative to zero.

4. A method according to claim 1, wherein step (d) is performed using the Fourier transform of said calibrated aortic pressure pulse waveform, calculating the flow wave components corresponding to each frequency component of the pressure wave separately, and combining the resulting waveforms to provide a derived flow velocity waveform.

5. A method according to claim 4, wherein in step (d), the flow wave component for each frequency component of the fourier transform is determined by reference to values for modulus and phase for each frequency within predefined age bands stored in a look up table.

6. A method according to claim 1, wherein a value for the imputed age which said predefined criteria are met is output.

**Patentansprüche**

1. Verfahren zum Bestimmen einer Wellenform einer Strömungsgeschwindigkeit in der Aorta mit den Schritten:

   a) nicht invasives Messen einer Wellenform eines Blutdruckimpulses an einer peripheren Stelle;
   b) Bestimmen eines kalibrierten im Wesentlichen simultanen systolischen und diastolischen Drukkes an der peripheren Stelle;
   c) Berechnen der kalibrierten Wellenform des Druckimpulses in der Aorta an der aufsteigenden Aorta unter Verwendung einer vorbestimmten Übertragungsfunktion;
   **gekennzeichnet durch** die Schritte:
   d) Berechnen der Wellenform der Strömungsgeschwindigkeit in der Aorta aus der Wellenform des Druckimpulses in der Aorta **durch** Bezugnahme auf vorbestimmte Werte für altersabhängige Impedanzbetrag und -phase;
   e) Bestimmen, ob die bei d) berechnete Wellenform vordefinierte Kriterien erfüllt, und falls nicht, iteratives Wiederholen des Schrittes d) unter Verwendung unterschiedlicher Werte für das Alter, bis die Wellenform die vordefinierten Kriterien erfüllt.

2. Verfahren nach Anspruch 1, wobei das Verfahren umfasst, dass ein eingegebener oder berechneter Zeitwert für den der Incisurpunkt für die kalibrierte Wellenform des Druckimpulses der Aorta gespeichert wird, und die vordefinierten Kriterien umfassen, dass an dem Incisurpunkt die Strömung im Wesentlichen Null beträgt.

3. Verfahren nach Anspruch 2, wobei die vordefinierten Kriterien umfassen, dass nach der Incisur die Strömung innerhalb einer vorbestimmten Spanne einer Spitzenströmung in Bezug auf Null bleibt.

4. Verfahren nach Anspruch 1, wobei der Schritt d) unter Verwendung der Fouriertransformation der kalibrierten Wellenform des Druckimpulses der Aorta durchgeführt wird, die Strömungswellenkomponenten gemäß jeder Frequenzkomponente der Druckwelle separat berechnet werden, und die resultierenden Wellenformen kombiniert werden, um eine abgeleitete Wellenform der Strömungsgeschwindigkeit bereitzustellen.

5. Verfahren nach Anspruch 4, wobei bei Schritt d) die Strömungswellenkomponente für jede Frequenzkomponente der Fouriertransformation durch Bezugnahme auf Werte für Betrag und Phase für jede Frequenz innerhalb vordefinierter Altersbänder, die in einer Nachschlagetabelle gespeichert sind, bestimmt wird.

6. Verfahren nach Anspruch 1, wobei ein Wert für das beigemessene Alter, bei dem die vordefinierten Kriterien erfüllt sind, ausgegeben wird.

**Revendications**

1. Procédé pour déterminer une forme d'onde de vitesse de courant aortique, comprenant les étapes consistant à :

   a. mesurer de manière non invasive à un site périphérique une forme d'onde d'impulsion de pression artérielle ;
   b. déterminer des pressions systolique et diastolique sensiblement simultanées étalonnées au site périphérique ;
   c. calculer la forme d'onde d'impulsion de pression aortique étalonnée à l'aorte ascendante en utilisant une fonction de transfert prédéterminée ;

**caractérisé en ce qu'**il comprend également les étapes consistant à :

d. calculer la forme d'onde de vitesse de courant aortique à partir de ladite forme d'onde d'impulsion de pression aortique en référence à des valeurs prédéterminées de module et de phase d'impédance dépendantes de l'age ; et

e. déterminer si la forme d'onde calculée dans (d) satisfait à des critères prédéfinis et si ce n'est pas le cas, répéter l'étape (d) de manière itérative en utilisant différentes valeurs pour l'age jusqu'auquel ladite forme d'onde satisfait aux critères prédéfinis.

2. Procédé selon la revendication 1, dans lequel ledit procédé comprend le stockage d'une valeur de temps entrée ou calculée pour le point d'incisure pour ladite forme d'onde d'impulsion de pression aortique, et lesdits critères prédéfinis tiennent compte qu'au point d'incisure, le débit est sensiblement égal à zéro.

3. Procédé selon la revendication 2, dans lequel lesdits critères prédéfinis tiennent compte qu'après l'incisure, le débit reste dans une marge prédéterminée de débit de crête par rapport à zéro.

4. Procédé selon la revendication 1, dans lequel l'étape (d) est effectuée en utilisant la transformée de Fourier de ladite forme d'onde d'impulsion de pression aortique étalonnée, en calculant les composantes d'onde de débit correspondant à chaque composante de fréquence d'onde de pression séparément, et en combinant les formes d'onde résultantes pour obtenir une forme d'onde de vitesse de courant dérivée.

5. Procédé selon la revendication 4, dans lequel, dans l'étape (d), la composante d'onde de débit pour chaque composante de fréquence de la transformée de Fourier est déterminée en référence à des valeurs de module et de phase pour chaque fréquence dans des bandes d'age prédéfinies stockées dans une table d'informations.

6. Procédé selon la revendication 1, dans lequel une valeur pour l'age théorique jusqu'auquel lesdits critères prédéfinis sont satisfaits est émis.

Fig 1.

Fig 2.

# Fig 3.